Europäisches Patentamt

European Patent Office

Office européen des brevets

(11) Veröffentlichungsnummer: **0 057 403**
**A1**

(19)

(12) **EUROPÄISCHE PATENTANMELDUNG**

(21) Anmeldenummer: **82100499.1**

(22) Anmeldetag: **26.01.82**

(51) Int. Cl.³: **A 61 K 33/40, A 61 K 7/48**

(30) Priorität: **30.01.81 US 230278**

(43) Veröffentlichungstag der Anmeldung: **11.08.82**
Patentblatt 82/32

(84) Benannte Vertragsstaaten: **AT BE CH IT LI LU NL SE**

(71) Anmelder: **Key Pharmaceuticals, Inc., 18425 N.W. 2nd Avenue, Miami, Florida 33169 (US)**

(72) Erfinder: **Keith, Alec D., 539 Beaumont Drive, State College, Pennsylvania 16801 (US)**

(74) Vertreter: **Werner, Hans-Karsten, Dr. et al, Deichmannhaus am Hauptbahnhof, D-5000 Köln 1 (DE)**

(54) Lecithin-Hydrogenperoxid-Zusammensetzung.

(57) Eine antibakterielle Zusammensetzung zur Behandlung von Akne enthält eine Mischung von etwa 0,25-15 Gewichts-% Lecithin und etwa 0,1-10 Gewichts-% Hydrogenperoxid in einem pharmazeutisch unbedenklichen Lösungsmittel, bezogen auf das Gesamtgewicht der Zusammensetzung.

EP 0 057 403 A1

## Lecithin-Hydrogenperoxid-Zusammensetzung

Die vorliegende Erfindung betrifft allgemein Zusammensetzungen, in denen Lecithin und Hydrogenperoxid in inniger Vermischung miteinander enthalten sind und durch die ein Mittel verfügbar gemacht wird, um Hydrogenperoxid in stabiler Form topisch einem Patienten zu verabreichen. Die Zusammensetzungen gemäß der vorliegenden Erfindung sind von besonderem Wert für die Behandlung von Akne.

Hydrogenperoxid ist seit langem für seine potente desinfizierende Wirkung bekannt. Die Vorteile dieser Wirksamkeit werden jedoch aufgehoben durch seine Instabilität (und die daraus sich ergebende kurze Lebensdauer während der Aufbewahrung) von wäßrigem Hydrogenperoxid. Wenn irgendein Hydrogenperoxid-Präparat auf die Haut gebracht wird, ist das Hydrogenperoxid der unmittelbaren Gefahr der Zersetzung durch Enzym-Prozesse ausgesetzt, etwa durch Katalase, die in menschlichen Geweben und in einer großen Mannigfaltigkeit von Bakterien vorhanden ist. Weiterhin verlieren Applikationen von wäßrigem Hydrogenperoxid auf der Haut rasch ihre Wirksamkeit, weil sie von der Haut ablaufen.

Die vorliegende Erfindung betrifft allgemein Zusammensetzungen, in denen Lecithin und Hydrogenperoxid in inniger Vermischung miteinander enthalten sind und durch die ein Mittel verfügbar gemacht wird, um Hydrogenperoxid in stabiler Form topisch einem Patienten zu verabreichen, insbesondere zur Behandlung von Akne. Die wesentlichen Bakterienstämme, die Akne und andere pathogene Hautvorgänge verursachen, können durch die

Applikation einer geringen Menge Hydrogenperoxid auf die Haut vollständig abgetötet oder in ihrem Wachstum wirksam gehindert werden. Dementsprechend macht die vorliegende Erfindung eine antibakterielle Zusammensetzung verfügbar, die eine Mischung von Lecithin und Hydrogenperoxid in einem pharmazeutisch unbedenklichen Lösungsmittel enthält. Die Hydrogenperoxid-Konzentration dieser Zusammensetzung beträgt 0,1 - 10 Gewichts-%, vorzugsweise etwa 4 - 8 Gewichts-% an Hydrogenperoxid, bezogen auf das Gesamtgewicht der Zusammensetzung. Die Gesamt-Konzentration an Lecithin beträgt etwa 0,25 - 15 Gewichts-%, vorzugsweise 1 - 2 %, bezogen auf das Gesamtgewicht der Zusammensetzung. Das bevorzugte Lösungsmittel für Zusammensetzungen gemäß der vorliegenden Erfindung ist Wasser. Da Zusammensetzungen gemäß der vorliegenden Erfindung häufig von Verbrauchern verwendet werden, können geeignete Trägermaterialien, Netzmittel oder Erweichungsmittel den Zusammensetzungen zugesetzt werden, um eine zusätzliche Befeuchtung der Haut und andere kosmetische Wirkungen herbeizuführen. Die Zusammensetzungen der vorliegenden Erfindung zeigen geringe Toxizität.

Die vorliegende Erfindung umfaßt auch eine Methode zur Behandlung von Akne durch Applizieren der Zusammensetzung der vorliegenden Erfindung, unter Verwendung von Wasser oder anderen pharmazeutisch unbedenklichen Lösungsmitteln, auf die Haut des Patienten.

Modifikationen und Anpassungen, die im Rahmen der hier offenbarten Erfindung möglich sind, sind für Fachleute aus der Beschreibung und den Ansprüchen ersichtlich.

## Beispiel I

Präparate gemäß der vorliegenden Erfindung wurden in bezug auf ihre Wirkung in vitro gegen zwei gewöhnliche Haut-Pathogene, S. aureus und P. acneus, untersucht. Scheibchen aus Filterpapier mit einem Durchmesser von 1,3 cm wurden mit einer Lecithin-Hydrogenperoxid-Mischung in Wasser gesättigt und in Petrischalen gelegt, die Kulturen der Test-Bakterien auf einem Medium auf Agar-Basis enthielten. In allen Fällen wurde der Grad der bakteriziden Wirkung durch die Messung der Gesamtbreite der Zone, in der um das Scheibchen herum eine Hemmung auftrat, bestimmt. In der folgenden Tabelle I sind die Ergebnisse dieser Tests aufgeführt, wobei die die Breite der Hemmzone bezeichnenden Zahlen ausschließlich des Durchmessers der Testscheibchen angegeben sind.

- 4 -

## Tabelle I

| Lecithin: (%) | $H_2O_2$: (%) | Breite der Hemmzone S.aureus (mm) | P.acneus (mm) |
|---|---|---|---|
| 2 | 0,5 | 10,6 | 17,3 |
| 2 | 0,5 | 11,6 | 12,4 |
| 1 | 1 | 12,0 | 13,6 |
| 1 | 1 | 12,9 | 16,9 |
| 2 | 1 | 11,5 | 14,9 |
| 2 | 1 | 12,7 | 14,4 |
| 4 | 1 | 11,5 | 17,3 |
| 4 | 1 | 13,7 | 16,2 |
| 6 | 1 | 13,0 | 14,9 |
| 8 | 1 | 12,6 | 14,9 |
| 8 | 2 | 15,4 | 21,6 |

## Beispiel II

Um zu bestimmen, ob das Lecithin die therapeutische Wirkung des Hydrogenperoxids in dem in Tabelle I wiedergegebenen Test hemmte oder maskierte, wurde wäßriges Hydrogenperoxid mit einer wäßrigen Zusammensetzung mit 4 % Lecithin und 1 % Hydrogenperoxid gemäß der vorliegenden Erfindung, die wie nachstehend angegeben formuliert wurde, verglichen.

|                                                     | Teile           |
|-----------------------------------------------------|-----------------|
| Soja-Lecithin, Alcolec L-100 (American Lecithin Co.) | 4,0             |
| Triethanolamin, 85 %, USP                           | 0,8             |
| Glycerin, 96 %, USP                                 | 5,0             |
| Mineralöl, USP                                      | 8,0             |
| Stearinsäure, USP                                   | 1,0             |
| Ethylenglycol-monostearat                           | 1,0             |
| Acetylierter Lanolinalkohol, Crodalan LA (Croda)    | 3,0             |
| Glyceryl-monostearat                                | 2,0             |
| Dimethicone                                         | 1,0             |
| Cetylakohol                                         | 0,2             |
| Imidazolidinyl-harnstoff                            | 0,5             |
| Methylparaben                                       | 0,3             |
| Propylparaben                                       | 0,2             |
| Carbopet 934 (B.F. Goodrich)                        | 0,2             |
| Magnesiumaluminiumsilikat                           | 0,2             |
| EDTA                                                | 0,02            |
| 10-proz. Hydrogenperoxid                            | 10,0            |
| Gereinigtes Wasser, USP                             | Q.s. zu 100 Teilen |

Die in der nachstehenden Tabelle II aufgeführten Zahlenwerte zeigen, daß kein signifikanter Unterschied hinsichtlich der in vitro-Hemmung von S. aureus zwischen 1-proz. wäßrigem Hydrogenperoxid und einer Zusammensetzung gemäß der vorliegenden Erfindung, die die gleiche Menge Hydrogenperoxid enthält, besteht.

0057403

## Tabelle II

|  | | Breite der Hemmzone | |
| --- | --- | --- | --- |
|  |  | Versuch 1 | Versuch 2 |
| 4 % Lecithin, 1 % $H_2O_2$ | | 3,5 | 3,5 |
| 3 % $H_2O_2$ | | 4,2 | 4,1 |
| 1 % $H_2O_2$ | | 3,5 | 3,6 |
| 0,3 % $H_2O_2$ | | 2,7 | * |
| 0,1 % $H_2O_2$ | | 2,0 | 2,0 |
| kein $H_2O_2$ | | --- | --- |

\* Test-Scheibchen fiel herunter, als die Schale gedreht wurde.

Wenngleich die vorstehend beschriebenen Plättchen-Tests nicht zwischen bakterizider und bakteriostatischer Wirkung unterscheiden, gilt diese Testmethode als ein Verfahren, das Aufschluß über die Signifikanz von Vorhersagen der in vitro-Aktivität gibt.

## Beispiel III

Es wurde gefunden, daß neben den Zusammensetzungen der Beispiele I und II die nachstehenden Zusammensetzungen mit höherer Potenz, bei denen weniger Lecithin eingesetzt wird, wirksam sind:

(A) Stärke: 7,5 %
Hydrogenperoxid 7,5 %
Lecithin 0,375 %
Entionisiertes Wasser Q.s.

(B) Stärke: 5,0 %
Hydrogenperoxid 5,0 %
Lecithin 0,25 %
Entionisiertes Wasser Q.s.

<u>P a t e n t a n s p r ü c h e</u>

1. Antibakterielle Zusammensetzung zur Behandlung von Akne, enthaltend eine Mischung von etwa 0,25 - 15 Gewichts-% Lecithin und etwa 0,1 - 10 Gewichts-% Hydrogenperoxid in einem pharmazeutisch unbedenklichen Lösungsmittel, bezogen auf das Gesamtgewicht der Zusammensetzung.

2. Zusammensetzung nach Anspruch 1, dadurch gekennzeichnet, daß die $H_2O_2$-Konzentration etwa 4 - 8 Gewichts-% beträgt.

3. Zusammensetzung nach Anspruch 1 oder 2, dadurch gekennzeichnet, daß das Lösungsmittel Wasser ist.

4. Zusammensetzung nach Anspruch 3, dadurch gekennzeichnet, daß sie etwa 1 Gewichts-% Lecithin enthält.

5. Zusammensetzung nach Anspruch 3, dadurch gekennzeichnet, daß sie etwa 1 bis 4 Gewichts-% Lecithin enthält.

6. Methode zur Behandlung von Akne, dadurch gekennzeichnet, daß die Zusammensetzung von Anspruch 3 auf die Haut eines Patienten, der an Akne leidet, aufgebracht wird.

VON KREISLER   SCHÖNWALD   EISHOLD   FUES
VON KREISLER   KELLER   SELTING   WERNER

C0057403

82 100 499.1
Key Pharmaceuticals, Inc.

PATENTANWÄLTE

Dr.-Ing. von Kreisler † 1973
Dr.-Ing. K. Schönwald, Köln
Dr.-Ing. K. W. Eishold, Bad Soden
Dr. J. F. Fues, Köln
Dipl.-Chem. Alek von Kreisler, Köln
Dipl.-Chem. Carola Keller, Köln
Dipl.-Ing. G. Selting, Köln
Dr. H.-K. Werner, Köln

Patentansprüche für Osterreich eingereicht am 27.Mai 1982 gemäss Regel
86(2).

DEICHMANNHAUS AM HAUPTBAHNHOF
D-5000 KÖLN 1    W/hg 4

25. Mai 1982

P a t e n t a n s p r ü c h e

1. Verfahren zur Herstellung einer antibakteriellen Zusammensetzung zur Behandlung von Akne, dadurch gekennzeichnet,
daß man 0,25 bis 15 Gew.-% Lecithin und 0,1 bis 10 Gew.-%
Wasserstoffperoxid zusammen mit einem pharmazeutisch unbedenklichen Lösungsmittel zu einer stabilen Zubereitung vermischt.

2. Verfahren gemäß Anspruch 1, dadurch gekennzeichnet, daß die
$H_2O_2$-Konzentration etwa 4 bis 8 Gew.-% beträgt.

3. Verfahren gemäß Anpspruch 1 oder 2, dadurch gekennzeichnet,
daß das Lösungsmittel Wasser ist.

4. Verfahren gemäß Anspruch 3, dadurch gekennzeichnet, daß etwa
1 bis 4 Gew.-% Lecithin verwendet werden.

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| XY | FR - A - 2 443 837 (QUINODERM LIMITED)<br><br>* Seite 1, Zeilen 29-33, Seite 9, Zeilen 1-15, Ansprüche 1-5 * | 1-6 |
| XY | GB - A - 1 539 771 (QUINODERM LIMITED)<br><br>* Seite 3, Zeilen 43-73 * | 1-6 |
| Y | HAGERS HANDBUCH DER PHARMAZEUTI-SCHEN PRAXIS, 4. Neuausgabe, Band 5, 1976, SPRINGER-VERLAG, Berlin, Heidelberg, New York, Seiten 471-473<br><br>* Seite 473, letzter Absatz (technische Anwendung) * | 1-6 |

**KLASSIFIKATION DER ANMELDUNG (Int. Cl.³)**

A 61 K 33/40
A 61 K   7/48

**RECHERCHIERTE SACHGEBIETE (Int. Cl.³)**

A 61 K

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

| X | Der vorliegende Recherchenbericht wurde für alle Patentansprüche erstellt. |
|---|---|

| Recherchenort | Abschlußdatum der Recherche | Prüfer |
|---|---|---|
| Den Haag | 14-04-1982 | BRINKMANN |

EPA form 1503.1   06.78